# EUROPÄISCHE PATENTSCHRIFT

(11) **EP 1 457 192 B1**
(45) Veröffentlichungstag und Bekanntmachung des Hinweises auf die Patenterteilung: **27.02.2008**
(21) Anmeldenummer: 04100994.5
(22) Anmeldetag: 11.03.2004
(51) Int. Cl.: A61Q 1/02, A61K 8/04, A61K 8/19, A61K 8/894

(54) **Dekorative Kosmetikprodukte mit Effektpigmenten**
Make-up comprising effect pigments
Composition cosmétique de maquillage contenant des pigments à effets

(30) Priorität: 14.03.2003 DE 10311302
(43) Veröffentlichungstag der Anmeldung: 15.09.2004
(73) Patentinhaber: Beiersdorf AG, 20245 Hamburg (DE)
(72) Erfinder: Lanzendoerfer, Ghita, 22087 Hamburg (DE); Stelling, Jessica, 22143 Hamburg (DE)

(56) Entgegenhaltungen:
- EP-A- 0 545 002
- EP-A- 0 972 512
- EP-A- 1 371 358
- EP-A- 1 424 372
- EP-A- 1 464 680
- WO-A-02/03951
- FR-A- 2 768 926

## Beschreibung

Die vorliegende Erfindung betrifft dekorative kosmetische und/oder dermatologische Zubereitungen, die Interferenzpigmente, die im folgenden unter Effektpigmente zusammengefasst werden, enthalten.

Effektpigmente bestehen aus plättchenförmigen Substraten, die mindestens eine Schicht eines optische dichteren Mediums besitzen, welche einen Teil der einfallenden Strahlung reflektieren, andere Teile transmittieren. Die an den Grenzflächen der Medien reflektierten Lichtwellen können interferieren: Sie löschen sich gegenseitig aus oder sie verstärken sich und erzeugen so ein schillerndes Farbenspiel oder winkelabhängige Farbänderungen.

Solche Farbeffekte sind bekannt aus der Natur, treten beispielsweise bei Perlmutt, Fischschuppen oder Perlen auf. Bei der Perle oder dem Perlmutt von Muscheln sind es die hauchdünnen, abwechselnden Schichten aus Kalk und Eiweiß, die den typischen Perlglanz durch die Mehrfachreflektion an den verschieden tief liegenden Schichten der Perle entstehen lassen. Bei auftreffenden Lichtstrahlen wirken die Calciumcarbonatschichten wie dünne, transparente Spiegel; sie reflektieren einen Teil des Lichtes, lassen jedoch einen Großteil passieren. Nach dem Durchdringen der Proteinschicht, trifft der Lichtstrahl dann auf die nächste Calciumcarbonatschicht und wird dort erneut geteilt: so entsteht nach dem Durchdringen des Lichtes vieler Grenzschichten der charakteristische Glanz von Perlen.

Interferenzpigmente können auch synthetisch hergestellt werden. Ihr Trägermaterial besteht klassisch aus Glimmer oder aus synthetischen Trägermaterialien wie z.B. Aluminiumoxid, Eisenoxiden, Siliziumdioxid oder Magnesiumfluorid. Letztere lassen sich in gleich bleibend hoher Qualität herstellen. Ihre Partikel zeichnen sich durch eine exakt definierte Geometrie, insbesondere Schichtdicke aus. Beschichtungen der Oberfläche dieser Plättchen mit Titandioxid oder anderen Metalloxiden mit höherem Brechungsindex als das Substrat steuern so den optischen Effekt der Pigmente. Sie eröffnen damit ein großes Spektrum neuer farblicher Gestaltungsmöglichkeiten. Zum Beispiel können Farben "floppen", das bedeutet, der Farbeindruck hängt von Blickwinkel des Betrachters ab: von oben gesehen erscheint eine Fläche grün, die seitlich betrachtet goldfarben wirkt. Solche Pigmente werden beispielsweise für Fahrzeug- und Gebrauchsgüterlackierungen eingesetzt.

Für die Interferenz-Farbe der Pigmente sind sowohl die Schichtdicke, der optisch dichteren Substanz als auch die Anzahl der aufgebrachten Schichten verantwortlich.

Eine anderer Weg zur Beschichtung von Glimmer- und Aluminiumplättchen besteht in der Anwendung eines CVD Prozesses, durch den Eisenoxid aufgebracht werden kann. Speziell im Rot- und Goldbereich zeigen diese Pigmente interessante Farbeindrücke und mit Aluminium als Träger bieten sie hohes Deckvermögen.

Für kosmetische Anwendungen bisher zugelassene Interferenzpigmente basieren auf Aluminium-, den verschiedenen Oxidationsstufen des Eisen, Siliziumdioxid-, Glimmer, Al₂O₃- und Natrium-Calcium-Borosilikat-Trägern. Diese können auf verschiedene Weise mit Fe₂O₃, Titandioxid (Anatas und Rutil), Karmin, Berliner Blau, Siliziumdioxid, Zinndioxid, Silber beschichtet sein. Auch mehrfach beschichtete Pigmente kommen zum Einsatz. Im folgenden werden Beispiele genannt, jedoch ohne limitierend zu sein:
o Al/SiO₂/Al/SiO₂/Al
o Cr/MgF₂/Al/MgF₂/Al
o MOS₂/SiO₂/Al/SiO₂/MoS₂
o Fe₂O₃/SiO₂/Al/SiO₂/Fe₂O₃
o Fe₂O₃/SiO₂/Al/SiO₂/Fe₂O₃
o Fe₂O₃/SiO₂/Fe₂O₃/SiO₂/Fe₂O₃
o MoS₂/SiO₂/Mica-Oxid/SiO₂/MoS₂
o Fe₂O₃/SiO₂/Mica-Oxid/SiO₂/Fe₂O₃

Allerdings ist es keineswegs einfach, diese geometrisch anspruchsvollen und ungewöhnlichen Pigmente in kosmetische Zubereitungen einzuarbeiten. Da der Erhalt Ihrer Geometrie, sowie der/den empfindliche(n) Beschichtung(en) aber zur Erreichung der gewünschten optischen Wirkung unbedingt erforderlich ist, darf diese durch den Formulierungsprozess keinen Schaden nehmen. Zur Formulierung kosmetischer Zubereitungen werden nämlich zum Mischen und auch zur Erreichung einer homogenen Zubereitung Verfahrensschritte durchlaufen, die die empfindlichen Interferenzpigmenzen einem hohen Maß an mechanischem Stress aussetzen. Im Besonderen fordern die weit verbreiteten Formulierungen auf Emulsionsbasis eine gute Homogenisierung, insbesondere dann, wenn große Mengen an Pigmenten zugegen sind wie in dekorativen Produkten. Je höher nämlich der Pigmentanteil ist, desto schwieriger ist es, stabile Formulierungen zu erhalten.

Außerdem sollen kosmetische Zubereitungen neben ihrer dekorierenden Wirkung auch noch weitere Anforderungen erfüllen, die oft mit Begriffen wie "kosmetische Eleganz" oder "angenehmes Hautgefühl" umschrieben werden. Damit ist im Grunde nichts anderes gemeint, als dass die Anwenderin oder der Anwender des Produktes durch die Anwendung keine zusätzlichen Beschwerungen seines Seins erfährt, sondern die Anwendung und damit das Produkt als rundum positiv und angenehm erlebt. All dies kann nur durch weitere Inhaltsstoffe erreicht werden. Solche können beispielsweise Silikonöle und Silikonelastomere sein, die allerdings wiederum erhöhte Anforderungen an die Künste des Herstellers derartiger Zubereitungen stellen.

Der Einsatz von festen elastomeren Polyorganosiloxanen oder Organopolysiloxanen, im folgenden als Siloxanelastomere bezeichnet, in kosmetischen Zubereitungen ist an sich bekannt und hat in den letzten Jahren an Bedeutung gewonnen. Diese Stoffe fanden neben dem Einsatz in Kosmetika Verwendung in Lebens- und Futtermittel, Arzneimitteln, Imprägniermitteln, Schmiermitteln und so fort. Siloxalelastomere sind teilweise oder vollständig vernetzt und weisen zumeist eine dreidimensionale Struktur auf. Sie sind erhältlich durch eine Reaktion von vinylendständigem Polymethylsiloxan und Methylhydrodimenthylsiloxan 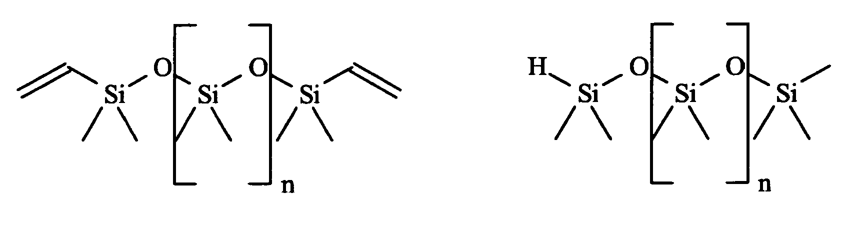 oder auch durch Reaktion von Hydroxy-endständigem Dimethylpolysiloxan und Trimethylsiloxyendständigem Methylpolysiloxan: 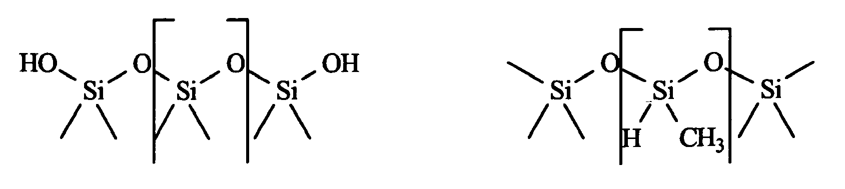 oder durch Reaktion eines α, ω- Dienes mit der Formel CH₂ =CH(CH₂)xCH=CH₂, mit x =1 - 20 und Methylhydrodimethylsiloxan ("New Developments in Silicone Elastomers for Skin Care", Michael Starch, Dow Corning, 2002).

Diese Siloxanelastomere werden beispielsweise zur Einstellung der rheologischen Eigenschaften einer Zubereitung eingesetzt. Beschrieben werden derartige Siloxanelastomere beispielsweise in der europäischen Patentschrift 295886 sowie der US-Patentschrift 5,654,362. In diesen Schriften ist auch die Natur der Siloxanelastomere näher beschrieben. Der Einsatz der Siloxanelastomere erfolgt in kosmetischen Zubereitungen insbesondere aufgrund ihrer angenehmen sensorischen Eigenschaften, die resultierenden Produkte werden als samtig, pudrig und/oder mattierend beschrieben. Daneben weisen sie stabilisierende Effekte auf hoch ölhaltige Formulierungen mit geringen Wassergehalten von höchstens 5 Gew.% auf. Bei der Formulierung der oben genannte Produkte stellt sich oftmals das Problem, daß die Siloxanelastomere mit anderen häufig eingesetzten Komponenten unverträglich sind, was zu unbefriedigender Langzeitstabilität der Produkte führt.

Die Schrift US 5738841 beschreibt zwar wasserfreie Pflege- oder Make-up-Produkte mit bestimmten Silikonkomponenten, aber nicht als wachsfreie Systeme und ohne den Einsatz der typischen kosmetischen Ester.

Die Schrift WO 9943297 beschreibt zwar Gele aus flüchtigen Kohlenwasserstoffen und Siloxanelastomeren, nicht aber in Form von Zubereitungen, die Interferenzpigmente enthalten. Die Schrift EP 790055 beschreibt zwar die Verwendung teilweise vernetzter elastomerer, fester Organopolysiloxane in Kombination mit einer Fettphase und flüchtigen Silikonölen zur Herstellung kosmetischer und/oder dermatologischer Zubereitungen um transfergehinderte Zubereitungen zu erhalten, nicht aber in Form von Zubereitungen, die Interferenzpigmente enthalten. Ferner kennt der Stand der Technik die Dokumente EP 0 545 002, EP 0 972 512, WO 02/03951, FR 2 768 926, doch konnten diese Schriften nicht den Weg zur vorliegenden Erfindung weisen, sowie die EP 1 464 680.

Ausgehend hiervon stellte sich die Aufgabe, Interferenzpigmente stabil in dekorative Zubereitungen einzuarbeiten und dabei den Zubereitungen die Vorteile einer angenehmen Sensorik, einem optimalen optischen Effekt der Pigmente und einer guten Deckkraft zu verleihen. Überraschend gelöst wird die Aufgabe gemäss Anspruch 1.

Es war für den Fachmann insbesondere nicht vorhersehbar, dass hohe Konzentrationen der Interferenzpigmente eingearbeitet und stabilisiert werden können.

Insbesondere wurde durch die erfolgreiche Herstellung solcher Zubereitungen das Problem gelöst, das insbesondere hochviskose (Viskosität > 9000 mPas) Gele jetzt homogen herstellbar sind und dabei gute Verteilbarkeit und Deckkraft z.B. auf dem Augenlid aufweisen. Dies war bisher nicht erreichbar, da durch den dafür erforderlichen Homogenisiervorgang das enthaltene Pigment stark zerstört wurde. Dies hat eine Ursache darin, daß das erfindungsgemäß verwendete Silikonelastomer so beschaffen ist, daß es die Oberfläche der Pigmentpartikel benetzt und das Pigment so gut in die Ölphase auch ohne das sonst erforderliche Anwenden hoher Scherraten einarbeitbar macht. Insbesondere von großem Vorteil ist es, daß zu solchen Gelen weiteres Öl (Gehalt von 0,5% bis 50%) zugegeben werden kann, ohne daß wie früher mit der dadurch sinkenden Viskosität der Zubereitung auch die Sedimentationsneigung ansteigt. Durch den hohen Gehalt an Interferenzpigmenten kann die Zubereitung stark deckend eingestellt werden, ohne daß zusätzlich Titandioxid eingesetzt werden müsste, welches früher zum Erreichen ausreichender Deckkraft erforderlich gewesen war und den Interferenz Effekt stark mindert.

Auf den sonst üblichen Einsatz von Wachsen, im Sinne von Estern einer langkettigen, aliphatischen Carbonsäure mit einem einwertigen, langkettigen Alkohol, kann verzichtet werden, wodurch die Zubereitungen bessere Fließeigenschaften aufweisen, insbesondere besonders gut aus Tuben entnehmbar sind. Zubereitungen des Standes der Technik weisen dagegen oft den Nachteil auf, nur widerspenstig bereit zu sein, ihre Tube zu verlassen.

Vorteile gegenüber Stand der Technik sind die gute Sensorik und leichte Verteilbarkeit der Formulierungen, die insbesondere bei der Applikation z.B. auf dem Augenlid von Vorteil sind, das gute Deckvermögen verbunden mit einem starken optischen Effekt (Perlmutt und /oder Farb-Flop), das die Verwendung als Eyeshadow, Blush oder auch Lippenstift begünstigt. Insbesondere lassen sich solche Effekte auf dem Augenlid gut darstellen, da durch die Krümmung des Lides bedingte winkelabhängige Eindrücke quasi "von selbst" entstehen. Weiterhin sind die so erhaltenen Formeln stabil und lassen sich trotz hoher Pigmentkonzentration gut aus den verschiedenen Packmitteln, z.B. einer Tube entnehmen.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Erfindungsgemäß vorteilhaft sind erfindungsgemäße kosmetische und/oder dermatologische Zubereitungen die dadurch gekennzeichnet sind, daß die Interferenzpigmente einen Träger aus Aluminium, Fe₂O₃, Siliziumdioxid, Glimmer, Al₂O₃ und/oder Natrium-Calcium-Borosilikat, sowie mindestens eine Beschichtung aus einer der Oxidationsstufen des Eisen, Titandioxid (rutil, anatase), Karmin, Berliner Blau, Siliziumdioxid, Zinndioxid, Silber, besonders bevorzugt Eisen(III)-oxid, Eisen(II, III)-oxid, Titandioxid, Siliziumdioxid, Zinndioxid und Silber aufweisen, so dass die Stärke der jeweiligen Beschichtung im Bereich 10 - 300 nm, besonders bevorzugt 20 bis 200 nm liegt und sowohl einfach, als auch mehrfach sein kann.

Dabei ist es erfindungsgemäß wenn in der erfindungsgemäßen kosmetischen und/oder dermatologischen Zubereitung der Massenanteil der Interferenzpigmente an der fertigen Zubereitung 10 bis 40%, besonders bevorzugt 12 bis 36%, ganz besonders bevorzugt 15 bis 30%, jeweils bezogen auf das Gesamtgewicht der Zubereitung beträgt.

Erfindungsgemäß vorteilhafte Ausführungsformen der erfindungsgemäßen kosmetischen und/oder dermatologischen Zubereitung sind dadurch gekennzeichnet, daß die flüchtigen Lipide gewählt werden aus der Gruppe der Silikonöle oder Paraffine, wobei Cyclohexane, Cyclopentane und Mischungen davon, sowie Isododecane erfindungsgemäß bevorzugt sind.

Es ist vorteilhaft im Sinne der vorliegenden Erfindung, wenn die erfindungsgemäße Zubereitung einen Massenanteil von 10 bis 40%, bezogen auf das Gesamtgewicht der Zubereitung, an flüchtigen Lipiden aufweist.

Erfindungsgemäß vorteilhafte Ausführungsformen der vorliegenden Erfindung sind dadurch gekennzeichnet, daß die Zubereitung einem Massenanteil von 30 bis 50%, bezogen auf das Gesamtgewicht der Zubereitung, an teilweise vernetzten elastomeren festen Organopolysiloxanen aufweist.

Vorzugsweise werden Siloxanelastomere gewählt aus den Gruppen der Siloxanelastomere, (a) die Einheiten R₂SiO und RSiO_{1,5} und/oder R3SiO_{0,5} und/oder SiO₂ enthalten, wobei die einzelnen Reste R jeweils unabhängig voneinander Wasserstoff, Alkyl wie beispielsweise Methy, Ethyl, Propyl oder Aryl wie beispielsweise Phenyl oder Tolyl, Alkenyl wie beispielsweise Vinyl bedeuten und das Gewichtsverhältnis der Einheiten R₂SiO zu RSiO_{1,5} im Bereich von 1:1 bis 30:1 gewählt wird; (b) die in Silikonöl unlöslich und quellfähig sind, die durch die Additionsreaktion eines Organopolysiloxans (1), das siliciumgebundenen Wasserstoff enthält mit einem vinylendständigem, aliphatischem Kohlenwasserstoff (2), erhältlich sind, wobei die verwendeten Mengenanteile so gewählt werden, daß die Menge des Wasserstoffes des Organopolysiloxans (1) oder der ungesättigten aliphatischen Gruppen des alph, omega- Diens (2) im Bereich von 1 bis 20 mol-% liegt, wenn das Organopolysiloxan nicht cyclisch ist und im Bereich von 1 bis 50 mol-% liegt, wenn das Organopolysiloxan cyclisch ist, verwendet werden. Besonders bevorzugt ist es, wenn das Organopolysiloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird. Ganz besonders bevorzugt ist es, wenn das Organopolysiloxanelastomer in Kombination mit unverzweigten bei Raumtemperatur flüssigen oder pastösen Silikonölen oder cyclischen Silikonölen oder deren Gemischen verwendet wird. Ganz außergewöhnlich bevorzugt ist es, wenn das Organopolysiloxanelastomer in Form eines Gels aus Organopolysiloxanelastomer und einer Lipidphase verwendet wird, wobei der Gehalt des Organopolysiloxanelastomers in dem Gel 3 bis 80 Gew.%, ganz außergewöhnlich stark bevorzugt 0,3 bis 60 Gew.% beträgt. Beispiele hierfür sind:
o DC 9040 Silikon Elastomer Blend (INCI: Cyclopentasiloxane (and) Dimethicone Crosspolymer (and) Cyclohexasiloxane), DC 9041 Silikon Elastomer Blend (INCI: Dimethicone (and) Dimethicone Crosspolymer), DC 9011 Silikone Elastomer Blend (INCI: Cyclopentasiloxane (and) PEG-12 Dimethicone Crosspolymer), Dow Corning (R)DC1-9852 Powder (INCI: Cyclomethicone (and) Dimethicone/ Vinyl Dimethicone Crosspolymer; proposed INCI) der Firma Dow Corning
o Gransil GCM-5 (INCI: Cyclopentasiloxane (D5) and Polysilikone-11), Gransil PM- Gel (INCI: Phenyl Trimethicone and Polysilikone-11), Gransil DMG-6 (INCI: Polysilikone-11 (and) Dimethicone) der Firma Grant Industries, Inc.
o Gel Base BSM-PT (lNCl: Cycolmethicone & Dimethicone& Petrolatum), Gel Base BSM 5 (INCI: Cycolmethicone & Dimethicone& Phenyl Trimethicone), Gel Base BSM PE (lNCl: Cycolmethicone & Dimethicone& Phenyl Trimethicone & Polyethylene) der Firma Arch Personal Care Products
o KSG 15 (INCI: Cyclopentsiloxane & Dimethicone/ Vinyl Dimethicone Crosspolymer), KSG 16 (lNCl: Dimethicone & Dimethicone/ Vinyl Dimethicone Crosspolymer) und KSG 18 (INCI: Phenyltrimethicone & Dimethicone/ Phenyl Vinyl Dimethicone Crosspolymer) der Firma Shin Etsu.

Eine andere Herstellungmethode, die Supensionsmethode liefert bei gleichen Edukten in einer Emulsion mit geringem Tensidgehalt stabilisiert sowohl feine Siloxanelastomerkügelchen in Wasser/Tensid Lösung, oder auch nach Sprühtrocknung das reine Siloxanelastomer in Pulverform. Als im Sinne der Erfindung vorteilhaft, jedoch nicht limitierend seien hier genannt:
o Dow Corning 9509 Silicone Elastomer Suspension (INCI: Dimethicone/ Vinyl Dimethicone Crosspolymer + C12 -14 Pareth- 12) der Firma Dow Corning
o Dow Corning 9506 Powder (lNCl: Dimethicone/ Vinyl Dimethicone Crosspolymer) der Firma Dow Corning.

Als Öle können alle unpolaren flüchtigen oder nicht flüchtigen Silikon- oder Kohlenwasserstofföle (Paraffine), einzeln oder in Mischungen, verwendet werden. Beispiel sind:
o Cyclomethicone, insbesondere Cyclotetrasiloxan (D4), Cyclopentasiloxan (D5) und Cyclohexasiloxan (D6), sowie deren Mischungen
o Dimethicone der allgemeinen Formel MDₙM mit n = 3 - 300
o Phenyltrimethicone, Bis-Phenylpropyl Dimethicone
o Flüchtige Kohlenwasserstoffe wie z.B. Isododecane

Vorteilhafte Filmbildner sind sowohl Dimeticonole und Abmischungen mit ihnen als auch Silikonpolymere mit Filmbildenden Eigenschaften. Beispiele sind:
o Dimiethiconole wie
   o DC 1501 (INCI: Cyclopentasiloxane and Dimethiconol), DC 3225C Formulation Aid (INCI: Cyclomethicone and Dimethicone Copolyol), DC Q2-1403 Fluid (lNCl: Dimethicone and Dimethiconol), DC 1401 (INCI: Cyclomethicone and Dimethconol) der Firma Dow Corning
o Trimethylsiloxysilikate, sowohl rein, als auch bereits vorgequollen. Beispiele sind.
   o Dow Corning 593 Fluid (INCI: Dimethicone (and) Trimethylsiloxysilicate) von Dow Coming
   o Wacker Belsil TMS 803 (INCI: Trimethylsiloxysilicate) von Wacker
o Silikoncopolymere, wie z.B.:
   o 2501 Cosmetic Wax (INCI: Dimethicone Copolyol), Mirasil Wax-S (INCI: Dimethiconol Stearate), Mirasil Wax-B (INCI: Dimethiconol Behenate), der Firma Rhodia
o Kohlenwasserstoffpolymere, wie z.B:
   o Performalene 400 (INCl: Polyethylene), New Phase Technologies
o Filmbildende Siliconether und Mischungen davon, wie z.B.:
   o 580 Wax (INCI: Stearoxy Trimethylsilane and Stearyl Alkohol) der Firma Dow Coming

Vorteilhafte Ausführungsformen der Erfindung enthalten Füllstoffe von 0 - 20%, bevorzugut von 0 - 10%, jeweils bezogen auf das Gesamtgewicht der Zubereitung.

Füllstoffe im Sinne der vorliegenden Erfindung sind partikuläre Substanzen, die in der Regel keinen Farbeffekt in der kosmetischen Formulierung erzeugen, in der sie eingesetzt werden. Ferner haben erfindungsgemäße Füllstoffe üblicherweise einen niedrigen Brechungsindex und daraus resultierend keine oder eine nur sehr geringe Deckkraft.

Der Stand der Technik kennt eine Reihe von Füllstoffen, welche z. B. als Trägermaterialien bei der Formulierung von Pudern oder als Viskositäts- und Sensorik-Modulatoren in Emulsionen oder wasserfreien Formulierungen dienen. Derartige Füllstoffe werden häufig auch eingesetzt, um mattierende Effekte auf der Haut zu erlangen oder um Sebum zu absorbieren.

Darüber hinaus beeinflußt der Einsatz von Füllstoffen im allgemeinen auch die Verteilbarkeit üblicher Formulierungen auf der Haut sowie die Gleichmäßigkeit eines möglichen Farbeffektes.

Die Füllstoffe im Sinne der vorliegenden Erfindung werden vorteilhaft aus der Gruppe der anorganische Füllstoffe gewählt, beispielsweise aus der Gruppe der Silikate.

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten.

Vorteilhaft im Sinne der vorliegenden Erfindung sind beispielsweise Schichtsilikate. Schichtsilikate (Phyllosilikate, Blattsilikate) sind (idealerweise) Silikat-Strukturen mit zweidimensional unendlichen Schichten aus [SiO₄]⁴⁻-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes lonenaustausch-Vermögen besitzen und Silizium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate, welche im Sinne der vorliegenden Erfindung eingesetzt werden können, sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Erfindungsgemäß vorteilhafte (Schicht-) Silikate sind insbesondere:
⇒ Talkum: Mg₃ [Si₄O₁₀] (OH)₂,
⇒ Kaolin: Al₂[Si₂O₅] (OH)₄
⇒ Montmorillonit: M⁺ Al [Si₂O₅](OH), auch Smektite genannt. Darunter fallen:
   - Bentonite = Montmorillonite mit Ca (Fuller Erden) oder Na (Wyoming Bentonite)
   - Hektorite: M⁺_{0,3}(Mg_{2,7}Li_{0,3})[Si₄O₁₀(OH)₂], worin M⁺ meist Na⁺ darstellt,
⇒ Glimmer (Mica), ein Alumosilikat, das leicht spaltbar ist und in tafeligen Kristallen vorliegt. Glimmer ist transparent bis durchscheinend und weist Perlglanz auf. Die wichtigste Form ist Muskovit: K Al₂ [AlSi ₃O₁₀] (OH, F)₂. Sericite ist eine Sonderform des Glimmers, die kleinere Plättchen als Muskovit aufweist.

Auch Siliziumoxide (SiO₂) sind vorteilhaft im Sinne der vorliegenden Erfindung zu verwenden. Erfindungsgemäß bevorzugt sind beispielsweise Aerosile (fumed Silica), welche hochdisperse Kieselsäuren mit häufig irregulärer Form sind, deren spezifische Oberfläche in der Regel sehr groß ist (200 - 400 m² / g) und mit Hilfe des Herstellverfahrens gesteuert werden kann. Aerosile werden auch bezeichnet als: Amorphous Silica Amorphous, Silicon Oxide Hydrate Silica, Amorphous Silicic Anhydride, Silicon Dioxide.

Erfindungsgemäß vorteilhafte Aerosile sind z. B. unter den folgenden Handlesnamen erhältlich: Aerosil 130 (Degussa Hüls), Aerosil 200 (Degussa Hüls), Aerosil 255 (Degussa Hüls), Aerosil 300 (Degussa Hüls), Aerosil 380 (Degussa Hüls), B-6C (Suzuki Yushi), CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), E-6C (Suzuki Yushi), Fossil Flour MBK (MBK), MSS-500 (Kobo), Neosil CT 11 (Crosfield Co.), Ronasphere (Rona/EM Industries), Silica, Anhydrous 31 (Whittaker, Clark & Daniels), Silica, Crystalline 216 (Whittaker, Clark & Daniels), Silotrat-1 (Vevy), Sorbosil AC33 (Crosfield Co.), Sorbosil AC 35 (Crosfield Co.), Sorbosil AC 37 (Crosfield Co.), Sorbosil AC 39 (Crosfield Co.), Sorbosil AC77 (Crosfield Co.), Sorbosil TC 15 (Crosfield Co.), Spherica (Ikeda), Spheriglass (Potters-Ballotini), Spheron L-1500 (Presperse), Spheron N-2000 (Presperse), Spheron P-1500 (Presperse), Wacker HDK H 30 (Wacker-Chemie), Wacker HDK N 20 (Wacker-Chemie), Wacker HDK P 100 H (Wacker Silicones), Wacker HDK N 20P (Wacker-Chemie), Wacker HDK N 25P (Wacker-Chemie), Wacker HDK S 13 (Wacker-Chemie), Wacker HDK T 30 (Wacker-Chemie), Wacker HDK V 15 (Wacker-Chemie), Wacker HDK V 15 P (Wacker-Chemie), Zelec Sil (DuPont).

Siliziumoxide lassen sich auch in sphärischer Form herstellen, wobei hier die spezifische Oberfläche kleiner ist als bei den Aerosilen, da die Teilchen größer und rund sind. Ein Beispiel hierfür sind die Ronaspheren (mittlerer Teilchendurchmesser < 3 µm) der Fa. Merck.
Weitere erfindungsgemäß bevorzugte Füllstoffe sind Siliziumdioxide, deren freien OH-Gruppen an der Teilchenoberfläche (ganz oder teilweise) organisch modifiziert sind.

Vorteilhaft sind z. B. die durch Addition von Dimethylsilyl-Gruppen erhältlichen Silica Dimethyl Silylate, wie beispielsweise Aerosil R972 (Degussa Hüls), Aerosil R974 (Degussa Hüls), CAB-O-SIL TS-610 (Cabot), CAB-O-SIL TS-720 (Cabot), Wacker HDK H15 (Wacker-Chemie), Wacker HDK H18 (Wacker-Chemie) und/oder Wacker HDK H20 (Wacker-Chemie).

Ferner vorteilhaft sind die durch Addition von Trimethylsily-Gruppen erhältlichen Silica Silylate (z. B. Aerosil R 812 (Degussa Hüls), CAB-O-SIL TS-530 (Cabot), Sipemat D 17 (Degussa Hüls), Wacker HDK H2000 (Wacker-Chemie)).

Ferner vorteilhaft im Sinne der vorliegenden Erfindung sind die durch Hydrolyse- und Kondensationsreaktionen von Methyltrimethoxysilane erhältlichen Polymethylsilsesquioxane, die ebenfalls eine runde Form besitzen und deren Teilchengrößenverteilung durch die Herstellung gesteuert werden kann.

Bevorzugte Polymethylsilsesquioxane werden beispielsweise unter den Handelsnamen Tospearl 2000 B von GE Bayer Silikones, Tospearl 145A von Toshiba, AEC Silicone Resin Spheres von A & E Connock sowie Wacker - Belsil PMS MK von der Wacker-Chemie angeboten.

Weiterer vorteilhafter Füllstoff im Sinne der vorliegenden Erfindung ist Bornitrid (siehe Fig. 2). Bornitrid ist isoelektronisch mit Kohlenstoff (d. h. es sind Graphit- und Diamantform möglich). Bornitrid zeichnet sich durch seine chemische Inertheit aus.

Vorteilhaft im Sinne der vorliegenden Erfindung sind beispielsweise die im folgenden aufgelisteten Bornitride:

| | |
|---|---|
| Handelsname | erhältlich bei: |
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bornitrid BNP | Wacker-Chemie |

Weitere vorteilhafte Füllstoffe im Sinne der vorliegenden Erfindung sind Carbonate, wie z. B. Magnesiumcarbonat (MgCO₃) und Calciumcarbonat (CaCO₃). Es ist insbesondere vorteilhaft im Sinne der vorliegenden Erfindung, die Carbonate als Füllstoffe in trockenen Pudern zu verwenden.

Die Füllstoffe im Sinne der vorliegenden Erfindung werden darüber hinaus vorteilhaft aus der Gruppe der organische Füllstoffe gewählt.

Erfindungsgemäß vorteilhafte organische Füllstoffe sind z. B. natürliche Polymere, wie Seidenpuder, mikrokristalline Cellulose und/oder Zinkstearate.

Vorteilhafte organische Füllstoffe sind ferner Stärke und Stärkederivate, wie:
⇒ Maisstärke Zea Mays (Amidon De Mais MST (Wackherr), Argo Brand Corn Starch (Corn Products), Pure-Dent (Grain Processing), Purity 21C (National Starch)),
⇒ Reisstärke (D.S.A. 7 (Agrana Stärke), Oryzapearl (Ichimaru Pharcos)),
⇒ Distarch Phosphate (Corn PO4 (Agrana Stärke), Corn PO4 (Tri-K)),
⇒ Sodium Corn Starch Octenylsuccinate (C* EmCap - Instant 12639 (Cerestar USA)),
⇒ Aluminium Starch Octenylsuccinate (Covafluid AMD (Wackherr), Dry Flo-PC (National Starch), Dry Flo Pure (National Starch), Fluidamid DF 12 (Roquette)),

Erfindungsgemäß bevorzugte organische Füllstoffe sind auch synthetische Polymere, d. h. Polymerpartikel, welche in der Zubereitung in Form von Feststoffen vorliegen, wie beispielsweise Polycarbonate, Polyether, Polyethylen, Polypropylen, Polyvinylchlorid, Polystyrol, Polyamide, Polyurethane, Polyacrylate und dergleichen mehr. Besonders vorteilhaft ist z. B. die Substanz mit der INCI-Bezeichnung *HDI* / *Trimethylol Hexyllactone Crosspolymer,* welche unter der Bezeichnung BPD-500/Plastic Powder D von der Firma Kobo erhältlich ist.

Ferner vorteilhaft im Sinne der vorliegenden Erfindung ist Nylon (Polyamid 6 und Polyamid 12), wie beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6), bzw. Polyamid 12- (auch: Nylon 12), Partikel. Polyamid 6 ist das aus ε-Aminocapronsäure (6-Aminohexansäure), oder ε-Caprolactam aufgebaute Polyamid [Poly(ε-caprolactam)], und Polyamid 12 ist ein Poly(ε-laurinlactam), aus ε-Laurinlactam. Vorteilhaft im Sinne der vorliegenden Erfindung sind beispielsweise Orgasol^{®} 1002 (Polyamid 6), Orgasol^{®} 2002 (Polyamid 12) und Orgasol^{®} 4000 (Polyamide 6/12), von der Firma Atofina.

Weitere vorteilhafte organische Füllstoffe sind:
⇒ PMMA: Polymethylmethacrylate
⇒ Polyethylene Spheres
⇒ Polyurethane

Effektpigment können nicht nur in Schichtdicke, Anzahl der Schichten, sowie ihrem Material variieren, sondern auch das plättchenförmigen Substrat, der Kern des Pigments, kann aus unterschiedlichen Materialien ausgewählt sein, so kann der Kern aus dabei prinzipiell aus allen plättchenförmigen Materialien bestehen, die senkrecht auftreffendes Licht ganz oder teilweise (üblicherweise zu mindestens 10%) reflektieren. In der Regel sind diese Materialien hochbrechend und haben üblicherweise einen Bechungsindex ≥2, vorzugsweise ≥2,4, sie können opak, semiopak oder transparent und in der Reflexion bzw. Transmission auch farbig sein. Dies triff in der Regel auf Metalle und Legierungen, z.B. Stahl, Kupfer und seine Legierungen wie Messing und Bronzen und insbesondere Aluminium und seine Legierungen wie Aluminiumbronze zu. Eine weitere Gruppe geeigneter Substratteilchen sind nicht-metallische Plättchen, die entweder selbst hochbrechend sind, oder niedrigbrechend und deshalb mit einer hochbrechenden Beschichtung versehen sind. Beispiele für besonders hochbrechende Materialien (selektiv oder nicht-selektiv absorbierende) sind z,B, plättchenförmige Metalloxide, -sulfide und -nitride wie vor allem plättchenförmiges, semiopakes α-Eisen(III)oxid (Hämatit), das mit Silicium, Aluminium oder Aluminium und Mangan dotiert sein kann, plättchenförmiges, opakes Eisen(II/III)oxid (Magnetit), Bornitrid und Graphitplättchen. Ebenfalls geeignet sind nichtabsorbierende (farblose), transparente Materialien, wie plättchenförmiges Bismutoxychlorid, Titandioxid und Zinkondioxidplättchen.
Beispiele für geeignete von sich aus nur niedrigbrechende Materialien sind vor allem silikatische Plättchen wie insbesondere hell bzw. weiße Glimmer, vorzugsweise nass vermahlener Muskovit, aber auch andere natürliche Glimmer, z.B. Phlogopit und Biotit, künstlicher Glimmer, Talk- und Glasschuppen, Magnesiumfluorid und Siliciumdioxidplättchen.
Bevorzugt wird das Substrat aus Siliciumdioxid, Aluminium, Eisenoxid oder Glimmer (Mica) augewählt.

Als hochbrechende Beschichtung für die niedrigbrechenden Materialien eignen sich hochbrechende Metalloxiode, Metallnitride und Metallsulfide wie Titan-, Zirkon-, Zink- und Zinnoxid, Bismuthoxichlorid, Eisenoxide, Cromoxid und llmenit, sowie reduzierte Titane mit Oxidationszahlen von <4 bis 2 enthaltene Titanverbindungen und seine Reduktionsprodukte sowie Eisen(III)oxid und ihre Schichtdicken liegen in der Regel zwischen 10 - 300 nm.(Irodin ® von Merck, Paliochrom von BASF und Mearlin® von Mearl)

Weiter eigenen sich als Beschichtungsmaterialien alle niedrigbrechenden, farblosen Substanzen, die filmartig und dauerhaft auf die Substratteilchen aufgebracht werden können, wobei hier entweder die hochbrechenden Substratteilchen verwendet werden können oder auch die aus niedrigbrechende Materialien bestehenden und bereits mit hochbrechenden Schichten umhüllten. Geeignet sind Metalloxide und Metalloxidhydrate wie Siliciumdioxid, Siliciumdioxidhydrat, Aluminiumoxid, Aluminiumoxidhydrat und deren Mischungen. Solche Schichten können eingelagerte, selektiv absorbierende Farbmittel enthalten, wobei prinzipiell Farbstoffe und Pigmente geeignet sind, die dauerhaft in die Schicht eingelagert werden können und deren Brechungsindex vorzugsweise nicht wesentlich über dem Brechungsindex des Schichtmaterials liegt (würde den Gesamtbrechungsindex erhöhen und den goniochromatischen Effekt mindern).
Neben niedrigbrechenden, anorganischen Pigmenten wie Cyanoferraten (z,B,: Fe₄ [Fe(CN)₆]₃), Granaten (z.B. Ca₃Cr₂Si₃O₁₂), Phosphaten (insbesondere Cobaltphosphaten, z.B. C₀₃(PO₄ₖ), Boraten und Ultramarin (Na4[AL₃Si₃O₁₂]s₃) eigenen sich vor allem organische Pigmente, da ihre Wechselwirkung mit Licht insbesondere auf Absorption beruht. Beispielhaft seinen hier folgende Pigmentklassen aufgeführt: Azo-, Diazo-, Antrachinon,- Phthalocyanin-, und Flavanthronpigmente, sowie Salze der natürlichen organischen Farbstoff (z.B.: Al-Ca-Lack der Karminsäure).
Besonders geeignet sind Farbstoffe, da sie im Gegensatz zu den Pigmenten nicht als Streuzentren wirken. Es können im Prinzip alle Farbstoffe in vorkommen, die für die dekorative Kosmetik zugelassen sind. Beispiele hierfür sind: natürliche organische Farbstoffe wie Karminsäure, Cyaninfarbstoffe, Di- und Triarylmethanfarbstoffe und Phenothiazinfarbstoffe.

Es sind hierbei sowohl einfache Beschichtungen, als auch Mehrfachbeschichtungen denkbar, wobei die einzelnen Schichten aus den verschiedenen Materialien gewählt sein können.

Zusätzlich können die Pigmente noch oberflächenmodifiziert sein. Hierunter wird eine mono- oder mehr molekulare Belegung der Pigmentoberfläche verstanden. Es können eine oder mehrere Substanzen entweder chemisch gebunden oder durch Physisorption an der Oberfläche haften. Im Sinne der Erfindung sind hier vor allem unpolare Oberflächenbelegungen von Vorteil wie Methicone (z.B.: Methylhydrogenpolysiloxane), Octylsilane (z.B.: Octyltrimethoxysilane, Trimethoxycaprylsilane), Octyldodecyl Myristate und ihre Mischungen.

| Substrat | Beschichtungen | | | | | | | | | | | | |
|---|---|---|---|---|---|---|---|---|---|---|---|---|---|
| | TiO₂ | Fe₂O₃ | Fe₃O₄ Ag | | Fe₄[Fe(CN₆)]₃ | Al | SnO₂ | Sn(IV)-Oxid | Mica | titanisiertes Mica | Karmine | SiO₂ | Beispiele |
| Al | | X | | | | | | | | | | X | Sicopearl Fantastico Pink und Gold, BASF/ |
| Al₂O₃ | X | | | | | | X | | | | | | Xirona Silver, Merck |
| Mica | X | | | | | | | | | | | X | Timiron Splendid blue, violet, green, gold, copper, Merck Flamenco AS; (Cadre/ Impag) |
| Mica | X | | | | X | | | | | | | | Duochrome Blue Green, Engelhard |
| Mica | X | X | | | | | | | | | | X | Colorona brilliant green, Merck |
| Mica | X | X | | | | | | | | | | | Timiron Fine Gold MP-20, Colorona Bright Gold, Colorona Red Gold, Timiron Karate Gold MP-24, Colorona Oriental Beige, Timiron Bronze MP-60, Flamenco Twilight Blue und Green sowie Desert Reflections-Serie von Engelhard |
| Mica | | X | | | | X | | | | | | | Colorona Glitter Orange, Merck |
| Mica | X | X | X | | | | | | | | | | Colorona Platina Gold, Merck |
| Mica | X | | X | | | | | | | | | | Colorona Platina Silver, Merck |
| Mica | | | X | | | | | | | | | | Colorona Blackstar, Blue, Red, Green, Gold, Merck |
| Mica | X | | | | X | | | | | | | | Dichrona BG, Merck |
| Mica | X | | | | | | | | | | X | | Dichrona RY, Merck |
| SiO₂ | X | | | | | | X | | X | | | | Xirona Caribian Blue, Merck |
| SiO₂ | X | | | | | | X | | | | | | Xirona Magic Mauve, Merck |
| SiO₂ | | X | | | | | | | | | | | Xirona Indian Summer, Merck |
| | | | | | | | | | | | | | |
| Fe₂O₃ | | X | | | | | | | | | | X | Sicopearl Fantastico Green |
| Fe₃O₄ | | | | | | | | | | X | | | Timica Nu Antique Silver 110AB, Engelhard |
| Fe₂O₃ + | | | | | | | | | | X | | | Timica Nu Antique Gold 212GB, Engelhard |
| Fe₃O₄ | | | | | | | | | | | | | |
| Calcium-Sodium Borosilicate | | | | X | | | | | | | | | Cosmetallic Scintillating Silver, Engelhard |

Es ist erfindungsgemäß von Vorteil, wenn in der erfindungsgemäßen Zubereitung zusätzlich lipophile Filmbildner, besonders bevorzugt Dimethicone Copolyol und/oder Dimethiconol enthalten sind.

Vorteilhafte Ausführungsformen der vorliegenden Erfindung zeichnen sich dabaei dadurch aus, daß die Zubereitung einem Massenanteil von 3 bis 12%, bezogen auf das Gesamtgewicht der Zubereitung, an lipophilen Filmbildnern aufweist.

Insbesondere ist es erfindungsgemäß, wenn die kosmetische und/oder dermatologische Zubereitung in Form eines Gels vorliegt.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

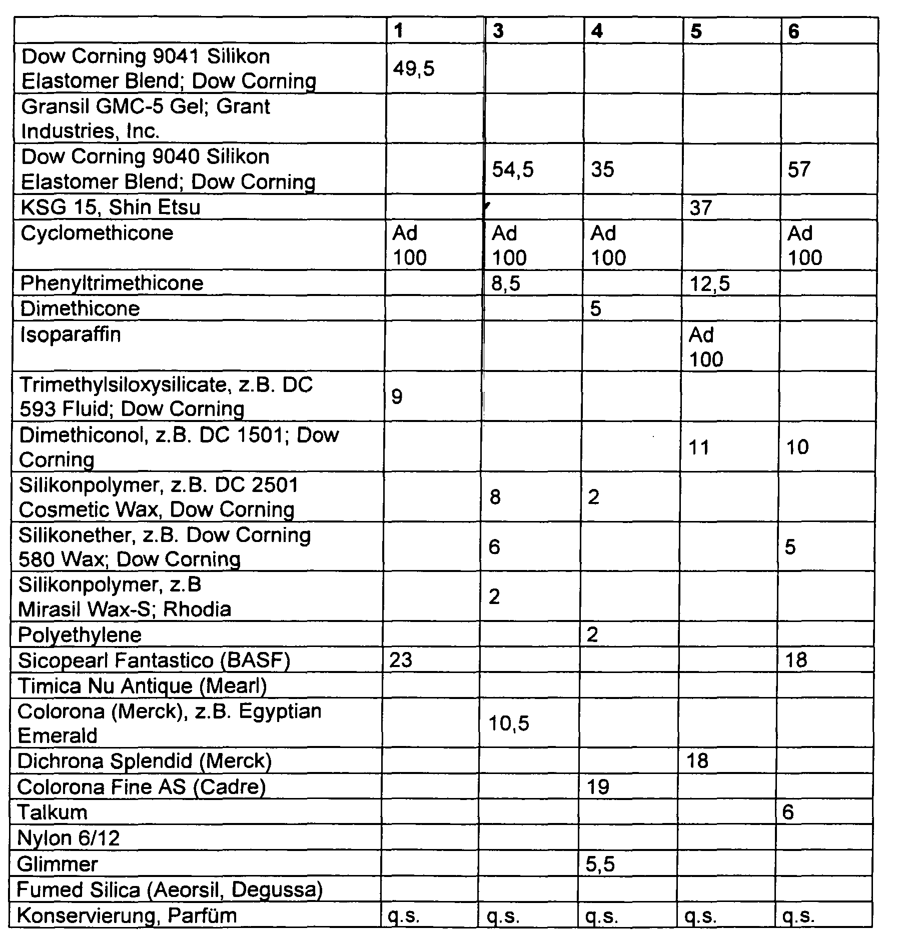

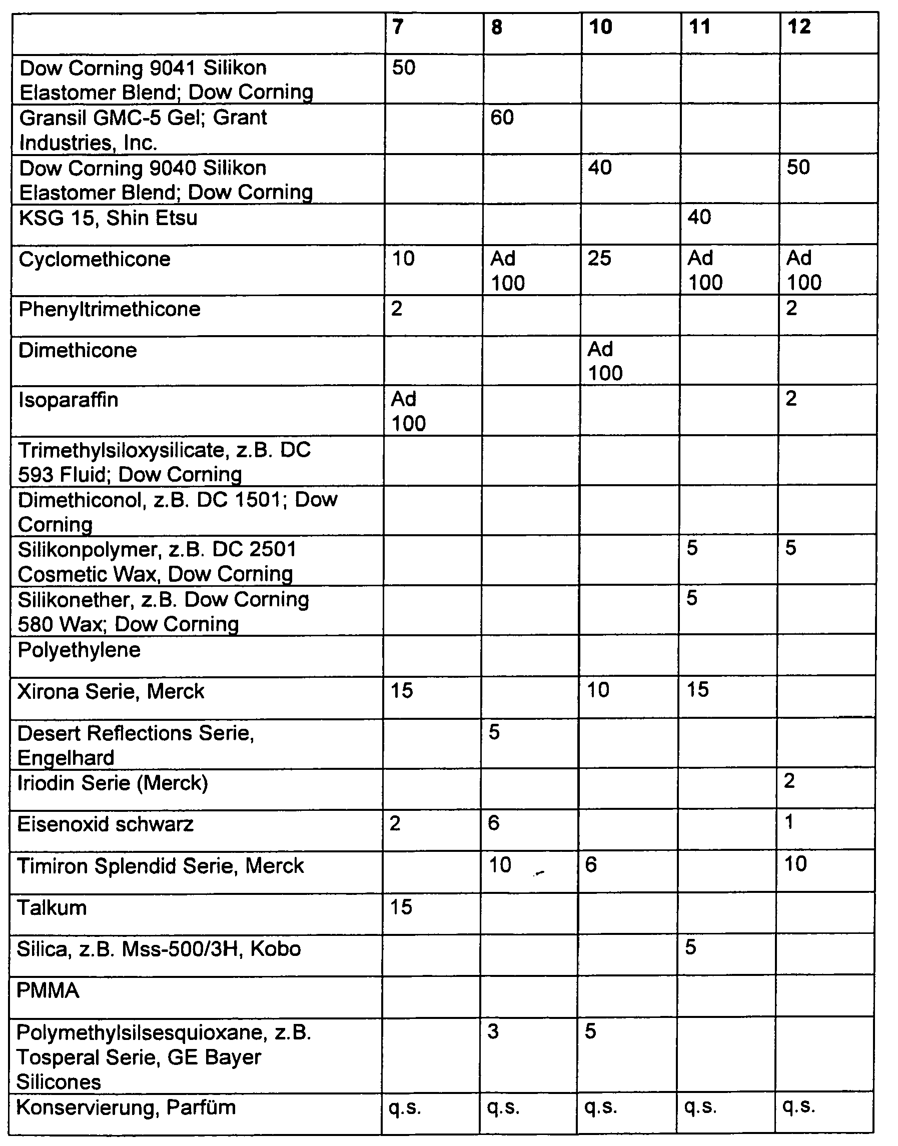

## Patentansprüche

1. Kosmetische und/oder dermatologische Zubereitung in Form eines Gels enthaltend teilweise vernetzte
elastomere feste Organopolysiloxane, flüchtige Lipide, Interferenzpigmente **dadurch gekennzeichnet, dass** der Massenanteil der Interferenzpigmentean der fertigen Zubereitung 10 bis 40%, besonders bevorzugt 12 bis 36%, ganz besonders bevorzugt 15 bis 30%, jeweils bezogen auf das Gesamtgewicht der Zubereitung beträgt, mit Ausnahme der Rezeptur
| | |
|---|---|
| verzweigtes Polydimethylorganosiloxan in Polydimethylsiloxan 6cs (gelierendes und mattifizierendes Agenz) | 20 g |
| Cyclopentadimethylsiloxan (Öl) | 29 g |
| Hydriertes Isoparaffin (Öl) | 10 g |
| Talkum (Füllstoff) | 6 g |
| Interferenzpigment | 10 g |
| Modifiziertes Hectorit (gelierender Ton) | Qs 100 g |

2. Kosmetische und/oder dermatologische Zubereitung nach Anspruch 1, **dadurch gekennzeichnet, dass** die Interferenzpigmente einen Träger aus Aluminium, Fe203, Siliziumdioxid, Glimmer, Al2O3 und/oder Natrium-Calcium-Borosilikat, sowie mindestens eine Beschichtung aus einer der Oxidationsstufen des Eisen, Titandioxid (rutil, anatase), Karmin, Berliner Blau, Siliziumdioxid, Zinndioxid, Silber, besonders bevorzugt Eisen(III)-oxid, Eisen(II, III)-oxid, Titandioxid, Siliziumdioxid, Zinndioxid und Silber aufweisen, so dass die Stärke der jeweiligen Beschichtung im Bereich 10 - 300nm, besonders bevorzugt 20 bis 200 nm liegt und sowohl einfach, als auch mehrfach sein kann.

3. Kosmetische und/oder dermatologische Zubereitung nach einem der vorangehenden Ansprüche **dadurch gekennzeichnet, dass** die flüchtigen Lipide gewählt werden aus der Gruppe der Silikonöle oder Paraffine, besonders bevorzugt sind Cyclohexane, Cyclopentane und Mischungen davon, sowie Isododecane.

4. Kosmetische und/oder dermatologische Zubereitung nach einem der vorangehenden Ansprüche **dadurch gekennzeichnet, dass** die Zubereitung einen Massenanteil von 10 bis 40%, bezogen auf das Gesamtgewicht der Zubereitung, an flüchtigen Lipiden aufweist.

5. Kosmetische und/oder dermatologische Zubereitung nach einem der vorangehenden Ansprüche **dadurch gekennzeichnet, dass** zusätzlich lipophile Filmbildner, besonders bevorzugt Dimethicone Copolyol und/oder Dimethiconol enthalten sind.

6. Kosmetische und/oder dermatologische Zubereitung nach einem der vorangehenden Ansprüche **dadurch gekennzeichnet, dass** die Zubereitung einem Massenanteil von 3 bis 12%, bezogen auf das Gesamtgewicht der Zubereitung, an lipophilen Filmbildnern aufweist.

## Claims

1. Cosmetic and/or dermatological preparation in the form of a gel comprising partly crosslinked, elastomeric, solid organopolysiloxanes, volatile lipids and interference pigments, **characterized in that** the mass fraction of the interference pigments as a proportion of the completed preparation is 10% to 40%, with particular preference 12% to 36%, with very particular preference 15% to 30%, based in each case on the overall weight of the preparation, with the exception of the following formula:
| | |
|---|---|
| Branched polydimethylorganosiloxane in polydimethylsiloxane 6cs (gelling and matting agent) | 20 g |
| Cyclopentadimethylsiloxane (oil) | 29 g |
| Hydrogenated isoparaffin (oil) | 10 g |
| Talc (filler) | 6 g |
| Interference pigment | 10 g |
| Modified hectorite (gelling clay) | qs 100 g |

2. Cosmetic and/or dermatological preparation according to Claim 1, **characterized in that** the interference pigments have a support of aluminium, Fe₂O₃, silicon dioxide, mica, Al₂O₃ and/or sodium calcium borosilicate and also have at least one coating of one of the oxidation states of iron, titanium dioxide (rutile, anatase), carmine, Prussian Blue, silicon dioxide, tin dioxide, silver, with particular preference iron(III) oxide, iron(II, III) oxide, titanium dioxide, silicon dioxide, tin dioxide and silver, such that the thickness of the respective coating is in the range 10-300 nm, with particular preference 20 to 200 nm, and can be both single and multiple.

3. Cosmetic and/or dermatological preparation according to any one of the preceding claims, **characterized in that** the volatile lipids are selected from the group of the silicone oils or paraffins, particular preference being given to cyclohexanes, cyclopentanes and mixtures thereof, and also isododecanes.

4. Cosmetic and/or dermatological preparation according to any one of the preceding claims, **characterized in that** the preparation has a mass fraction of volatile lipids of 10% to 40%, based on the overall weight of the preparation.

5. Cosmetic and/or dermatological preparation according to any one of the preceding claims, **characterized in that** lipophilic film formers, with particular preference dimethicone copolyol and/or dimethiconol, are additionally included.

6. Cosmetic and/or dermatological preparation according to any one of the preceding claims, **characterized in that** the preparation has a mass fraction of lipophilic film formers of 3% to 12%, based on the overall weight of the preparation.

## Revendications

1. Préparation cosmétique et/ou dermatologique sous forme d'un gel contenant des organopolysiloxanes solides élastomères partiellement réticulés, des lipides volatils, des pigments interférents, **caractérisée en ce que** la proportion pondérale des pigments interférents par rapport à la préparation finie est de 10 à 40 %, d'une manière particulièrement préférée de 12 à 36 %, d'une manière tout particulièrement préférée de 15 à 30 %, dans tous les cas par rapport au poids total de la préparation, à l'exception de la formulation
| | |
|---|---|
| Polydiméthylorganosiloxane ramifié dans du polydiméthylsiloxane 6cs (agent gélifiant et matifiant) | 20 g |
| Cyclopentadiméthylsiloxane (huile) | 29 g |
| Isoparaffine hydrogénée (huile) | 10 g |
| Talc (matière de charge) | 6 g |
| Pigment interférent | 10 g |
| Hectorite modifiée (argile gélifiante) | qs 100 g |

2. Préparation cosmétique et/ou dermatologique selon la revendication 1, **caractérisée en ce que** les pigments interférents comprennent un support constitué d'aluminium, de Fe₂O₃, de dioxyde de silicium, de mica, d'Al₂O₃ et/ou de borosilicate de sodium et de calcium, ainsi qu'au moins un revêtement constitué de l'un des degrés d'oxydation du fer, de dioxyde de titane (rutile, anatase), de carmin, de bleu de Prusse, de dioxyde de silicium, de dioxyde d'étain, d'argent, d'une manière particulièrement préférée d'oxyde de fer(III), d'oxyde de fer(II, III), de dioxyde de titane, de dioxyde de silicium, de dioxyde d'étain et d'argent, de telle sorte que l'épaisseur de chaque revêtement soit comprise dans la plage de 10 à 300 nm, d'une manière particulièrement préférée de 20 à 200 nm, et puisse être tant simple que multiple.

3. Préparation cosmétique et/ou dermatologique selon l'une des revendications précédentes, **caractérisée en ce que** les lipides volatils sont choisis dans le groupe des huiles de silicone ou des paraffines, et on préfère en particulier les cyclohexanes, les cyclopentanes et leurs mélanges, ainsi que les isododécanes.

4. Préparation cosmétique et/ou dermatologique selon l'une des revendications précédentes, **caractérisée en ce que** la préparation présente une proportion pondérale des lipides volatils de 10 à 40 % par rapport au poids total de la préparation.

5. Préparation cosmétique et/ou dermatologique selon l'une des revendications précédentes, **caractérisée en ce qu'**elle contient en outre des agents filmogènes lipophiles, d'une manière particulièrement préférée du diméthicone-copolyol et/ou du diméthiconol.

6. Préparation cosmétique et/ou dermatologique selon l'une des revendications précédentes, **caractérisée en ce que** la préparation comprend une proportion pondérale d'agents filmogènes lipophiles de 3 à 12 % par rapport au poids total de la préparation.
